# EUROPEAN PATENT APPLICATION

(11) **EP 4 043 018 A1**
(43) Date of publication of application: **17.08.2022**
(21) Application number: 21156284.8
(22) Date of filing: 10.02.2021
(51) Int. Cl.: A61K 31/713, A61P 13/12

(54) **COMPOSITION AND METHOD FOR REDUCING OXALATE LEVELS IN PATIENTS RECEIVING MAINTENANCE DIALYSIS**

(71) Applicant: Charité - Universitätsmedizin Berlin, 10117 Berlin (DE)
(72) Inventor: Knauf, Felix, 10709 Berlin (DE); Pfau, Anja, 12161 Berlin (DE)
(74) Representative: Hertin und Partner Rechts- und Patentanwälte PartG mbB

(57) **Abstract**

The invention relates to a composition comprising Lumasiran and/or Nedosiran for use in the treatment or prevention of an oxalate-related disorder in a patient receiving dialysis treatment who does not suffer from Primary Hyperoxaluria 1-3 (PH), preferably a cardiovascular disease. In further aspects of the invention, the composition comprising Lumasiran and Nedosiran diminishes or blocks hepatic oxalate production in non-PH dialysis receiving patients to prevent or treat cardiovascular events, sudden cardiac death, congestive heart failure, and excess mortality in said patients. The invention further relates to administration, a method for preparing the composition comprising Lumasiran and/or Nedosiran and a kit comprising Lumasiran and/or Nedosiran and instructions for its use.

## Description

The invention relates to a composition comprising Lumasiran and/or Nedosiran for use in the treatment or prevention of an oxalate-related disorder in a patient receiving dialysis treatment who does not suffer from Primary Hyperoxaluria 1-3 (PH), preferably a cardiovascular disease. In further aspects of the invention, the composition comprising Lumasiran and Nedosiran diminishes or blocks hepatic oxalate production in non-PH patients receiving dialysis to prevent or treat cardiovascular events, sudden cardiac death, congestive heart failure, and excess mortality in said patients. The invention further relates to administration, a method for preparing the composition comprising Lumasiran and/or Nedosiran and a kit comprising Lumasiran and/or Nedosiran and instructions for its use.

### BACKGROUND OF THE INVENTION

Oxalate is absorbed into the human body via the diet or a by-product of the human metabolism produced in the liver. Oxalate cannot be degraded but is excreted from the human body via two different routes: the kidney and the intestine.¹ It is of essential importance that excretion of oxalate is being maintained via the urine and through the intestinal lumen throughout life since excess oxalate levels are potentially toxic to the human system. Physical manifestations of primary and secondary hyperoxaluria are two distinct oxalate related conditions characterized by, for example, abnormal urinary oxalate excretion. Primary hyperoxaluria (PH, type 1-3) is an inherited genetic disorder with defective enzyme activities in which there is a pathological overproduction of oxalate in the liver. The long-term consequences of this massive oxalate load are kidney stones, renal insufficiency and even the need for dialysis, as well as diffuse deposits of oxalate in internal organs and tissues. PH type 1 is the most common and severe form (80% of all PH types): it can be lethal even in childhood and liver-kidney transplantation has been considered the only causal therapy.² Secondary hyperoxaluria can be caused by a number of factors, such as increased dietary intake of oxalate or precursors of oxalate or changes in intestinal absorption or excretion of oxalate or fat and alterations in the intestinal microbiota or genetic variations in intestinal or tubular oxalate transporter protein expression.

Oxalate-related chronic kidney diseases due to secondary hyperoxaluria is also an increasing worldwide health problem. It is characterized, for example, by progressively increasing oxalate concentrations in the urine. Elevated oxalate concentrations bear risks for the formation of calcium oxalate crystals in the distal tubules and the connecting duct with subsequent formation of calcium oxalate deposits or calcification of the kidney, also leading to kidney stones and declining kidney function. When the calcium oxalate load exceeds the excretory capacity of the kidneys, calcium oxalate begins to be deposited in all body fluids, bones, and soft tissues.

In recent years, new drug therapies have been established that use the principle of RNA interference to specifically suppress hepatic formation of oxalate at the mRNA level. Currently, two compounds are in development: Lumasiran (ALN-GO1) from Alnylam for the treatment of PH type 1 and Nedosiran (DCR-PHXC) from Dicerna Pharmaceuticals for the treatment of all 3 forms of PH. Lumasiran blocks the translation of hydroxyacid oxidase HAO1 (glycolate oxidase, GO).

Lumasiran is currently being tested in clinical trials for its ability to inhibit HAO1 in PH1 patients. PH1 is an autosomal recessive disease caused by mutations in the AGXT gene, encoding the liver alanine-glyoxylate aminotransferase (AGT) enzyme. These mutations result in a reduction in the amount or function of the enzyme, preventing the breakdown of glyoxylate. Glyoxylate accumulates and is reduced to glycolate, which in turn is converted to oxalate by HAO1. Encouraging data from lumasiran were already officially published in June 2018 at the OxalEurope meeting. In June 2020, clinical phase III data were presented at the ERA-EDTA Congress showing that in 26 PH1 patients with a glomerular filtration rate (GFR) ≥30 ml/min/1.73 m² of the kidney, administration of lumasiran resulted in an approximately 65% decrease in urinary oxalate from baseline and a 39.8% decrease in plasma oxalate (ILLUMINATE-A study). In normally functioning kidneys, the GFR is 90-130 milliliters per minute. Values lower than 60ml/min indicate a severe renal damage. A marketing authorization application has been submitted to both the EMA (European Medicines Agency) and the FDA (Food and Drug Administration). In the meantime, the drug has been officially approved by the EMA and the FDA since the end of 2020. The marketing authorization applications and the indication of Lumasiran refer exclusively to patients suffering from PH-1; all studies were conducted exclusively in PH-1 patients or in Phase I in healthy volunteers.

Nedosiran blocks the translation of liver-specific lactate dehydrogenase-A (LDH-A.).³ Studies on Nedosiran exclusively include patients suffering from PH 1-3. Currently, the product is being evaluated in 2 Phase II studies (NCT03847909, NCT04580420) and one Phase III study (NCT04042402). Most recently, preliminary positive results were published in March 2020, reporting a relevant reduction in urinary oxalate concentrations (reflecting endogenous oxalate load).⁴

Oxalate concentrations are increased not only in patients suffering from primary or secondary hyperoxaluria, but also in other patients receiving hemodialysis or peritoneal dialysis (=non PH dialysis receiving patient). Non-PH dialysis receiving patients are hardly able to excrete oxalate via the kidney. It is a serious but so far overlooked problem that these patients still continue to produce oxalate in the liver and also receive oxalate from diet. To date, the clinical significance of oxalate in the non-PH dialysis receiving patient population has been unclear, although numerous papers have reported evidence of a link between oxalate and inflammation as well as the progression of chronic renal failure.^{5,6}

Clinical evidence supporting an association between an oxalate load in non-PH dialysis receiving patients and health outcomes and risks for secondary diseases is lacking to date. In addition, dialysis patients are not yet treated and monitored for high oxalate levels.

This is an unmet and urgent medical need in the health care of non-PH dialysis receiving patients.

### SUMMARY OF THE INVENTION

In light of the prior art, the technical problem underlying the invention was the provision of an alternative or improved means for treating or preventing medical conditions associated with oxalate concentrations in serum and plasma in a patient receiving dialysis treatment who does not suffer from Primary Hyperoxaluria 1-3 (PH).

The technical problem may also be viewed as the provision of means for the treatment and/or reduction of risk of medical conditions associated with plasma or serum oxalate concentrations at therapeutically relevant levels or oxalate deposits in organs or tissues. The technical problem may also be viewed as the provision of means for the prevention and/or treatment of oxalate or oxalate deposit related disorders in non PH patients receiving dialysis, more particular oxalate related cardiovascular disorders, oxalate deposit related cardiovascular disorders, vascular calcification, and excess mortality.

This problem is solved by the features of the independent claims. Preferred embodiments of the present invention are provided by the dependent claims.

The invention therefore relates to a composition comprising Lumasiran and/or Nedosiran for use in the treatment or prevention of an oxalate-related disorder in a patient receiving dialysis treatment who does not suffer from Primary Hyperoxaluria 1-3 (PH).

According to the present invention provided herein, a composition comprising Lumasiran and/or Nedosiran as well as a drug product comprising said composition have a therapeutically relevant effect on oxalate levels in serum or plasma in a patient receiving dialysis treatment who does not suffer from Primary Hyperoxaluria, further referred to as non-PH dialysis patients. Particularly disorders are considered which are associated with oxalate related calcium-oxalate deposition, such as calcium-oxalate deposition related disorder involving therapeutic relevant levels of oxalate in plasma or serum.

The invention also relates to the combined administration of a therapeutically effective amount of Lumasiran and a therapeutically effective amount of Nedosiran in such treatment or prevention. Therapeutic relevant levels of oxalate in the plasma and serum can lead to the formation and deposition of calcium oxalate complexes in the body. Non-PH dialysis patients (almost) excrete no oxalate via the kidney, but continue to produce oxalate in the liver. It has been shown difficult in the art to elucidate the significance of oxalate in non-PH dialysis patients and to identify compositions that have a therapeutic effect on oxalate levels and formed oxalate deposits in non-PH patients receiving dialysis by blocking or diminishing oxalate production. If such a therapeutic effect can be achieved, it may lower serum or plasma oxalate concentrations in non-PH patients receiving dialysis, thereby delaying or interrupting the formation of oxalate deposits or aiding in the elimination of oxalate deposits and treating or preventing associated diseases.

The present invention provides such a significance and such an effect which are further described below. The inhibition or reduction of endogenous hepatic oxalate production and thus of the oxalate concentration in non-PH dialysis patients via Lumasiran and/or Nedosiran offers a potential new therapeutic strategy to reduce the high mortality risk of dialysis patients, as described herein. The present invention provides means having a surprisingly beneficial effect on non-PH dialysis patients' survival in oxalate-associated mortality.

Therefore, use of the pharmaceutical composition is provided herein particularly in the treatment of a disease associated with therapeutically relevant serum and plasma oxalate concentrations, oxalate crystal deposition, and impaired non-PH renal function. Since a therapeutically relevant effect on oxalate concentration in serum and plasma and oxalate deposits may take some time during treatment with Lumasiran and/or Nedosiran, a treatment period of days, weeks, several months to several years or lifelong is intended in non-PH dialysis patients.

As described at length herein, the composition comprising Lumasiran and/or Nedosiran represents a novel approach towards developing an effective measure to treat or prevent an oxalate-related disorder in a non-PH patient receiving dialysis.

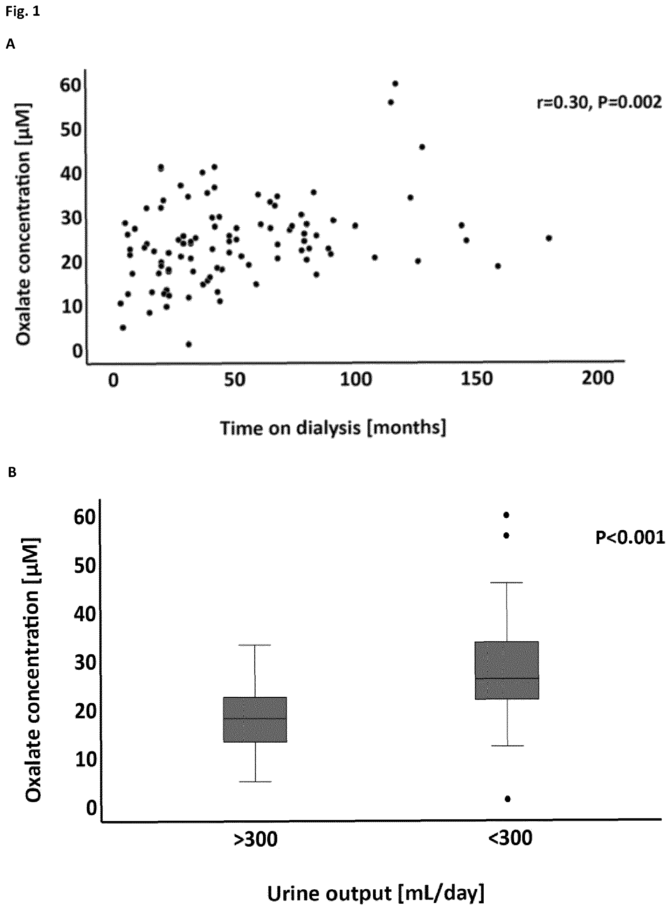

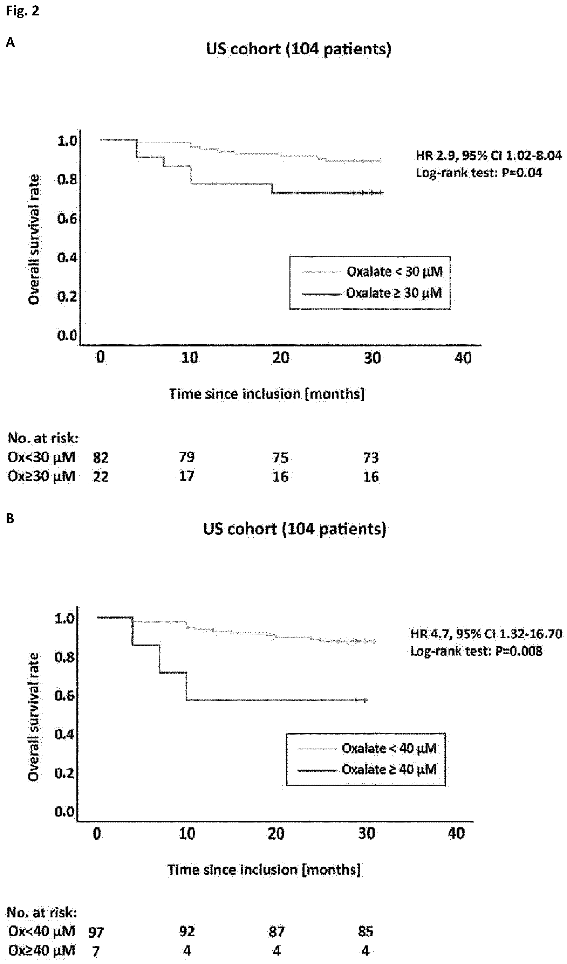

The novel medical use and indication described herein enables treatment of a disease, for which the medical community is unaware of oxalate-related disorder in a non-PH patient receiving dialysis and for which patients are in desperate need of means to prevent and treat oxalate-related disorder and mortality. At present, said patients are not at all or not appropriately treated as no effective medication may be available for the patient group described herein. As evident from the experimental support provided herein (Example 1, Figure 1A), increased plasma oxalate levels are significantly associated with time on dialysis (Figure 1A) and patient urinary output. As evident in the examples, oxalate concentrations in dialysis-dependent patients are unexpectedly associated with an increased risk of death (Figure 2A). In Figure 2B, high serum oxalate levels are further associated with death due to heart failure as well as sudden cardiac death in the 4D patient cohort. The combinations of these properties could not have been predicted by a skilled person and therefore relate to non-obvious, special technical features of the invention.

In one embodiment, Lumasiran inhibit mRNA translation of glycolate oxidase in a target cell, preferably a hepatic cell. Lumasiran reduce plasma or serum oxalate concentration by each dose administered.

In one embodiment, Nedosiran inhibit mRNA translation of liver-specific LDH-A in a target cell, preferably a hepatic cell. Nedosiran reduce plasma or serum oxalate concentration by each dose administered. In one embodiment, reduction of oxalate concentration is amplified when Lumasiran and/or Nedosiran are administered in the inventive composition.

In one embodiment, the reduction of oxalate concentration is determined by quantifying oxalate levels in plasma and/or serum, from non-PH patient, preferably at the time of receiving dialysis treatment. In one embodiment, oxalate in plasma and serum from non-PH patient receiving dialysis is determined as described in the Examples.

In one embodiment, prior to initiating treatment or prevention of non-PH1 patients receiving hemodialysis, a blood sample is collected prior to dialysis therapy at the first appointment after a long interval. Said blood sample is preferably used for determining oxalate concentrations in plasma and serum according to the pre-analytics and by means of an oxidase assay described in the Examples. More details for sample handling and measurement are described in the Examples.

Lumasiran and Nedosiran are tested in clinical studies to treat patients suffering from Primary hyperoxaluria (PH, type 1-3), a genetically determined metabolic disease in which pathological overproduction of oxalate occurs in the liver, resulting in a massive oxalate load in the patient. Lumasiran by Alnylam (patents US202002062581; US20200165616) and Nedosiran by Dicerna (WO2015100436) are investigational, subcutaneously administered RNA interference (RNAi) therapeutics that block mRNA translation of proteins responsible for hepatic oxalate production. Lumasiran inhibits translation of glycolate oxidase, and nedosiran blocks translation of liver-specific LDH-A. The marketing authorization applications and the field of application of Lumasiran and Nedosiran exclusively cover patients suffering from PH 1-3. Compared to placebo, Lumasiran (ILLUMINATE-A study, NCT03681184) reduced urinary oxalate by 53.5% on average. For Nedosiran (PHYOX2/3; NCT03847909), more than 50% of patients reached normal urinary oxalate levels.

The present invention therefore represents a tailored intervention for reducing oxalate concentrations by blocking glycolate oxidase mRNA translation in combination with inhibiting mRNA translation of liver-specific LDH-A in non-PH dialysis patients or only one of each therapeutic.

In some embodiments, oxalate-related disorders comprise cardiovascular disease in a non-PH patient receiving dialysis treatment.

As shown below, by conducting a thorough clinical study, the inventors surprisingly demonstrated an association between oxalate concentrations in serum and plasma and cardiovascular diseases, and in relation thereto, found a beneficial medical use of the pharmaceutical composition comprising Lumasiran and/or Nedosiran.

Beneficial effects for oxalate-related cardiovascular diseases in a non-PH patient receiving dialysis treatment can be achieved through the reduction of oxalate concentration at therapeutic levels by Lumasiran and/or Nedosiran.

In one embodiment, it is an object of the invention to provide composition for the prevention or treatment of cardiovascular diseases caused by oxalate in a non-PH patient receiving dialysis treatment. The inventors surprisingly found in two independent patient cohorts (Example 1 and 2) that higher plasma or serum concentrations of oxalate in the non-PH patient dialysis patients were associated with cardiovascular diseases, preferably sudden cardiac death. Calcium oxalate was identified as the primary contributor to vascular calcification in the non-PH dialysis patient.

The present invention is related to a risk of developing a cardiovascular disease in non-PH dialysis patient with therapeutically relevant oxalate levels in serum and plasma. The composition provided by the present disclosure may be capable of preventing, delaying or stopping the development of oxalate-related cardiovascular diseases.

In one embodiment, associations between serum oxalate and myocardial infarction and stroke are also consistent with the overall findings for the composite events, heart failure and sudden cardiac death. Oxalate concentrations are surprisingly mechanistically involved. As evident in the example 1, one fifth of all patients had oxalate plasma concentrations ≥30 µM. A skilled person could not have been expected that high serum oxalate levels were associated with excess mortality due to heart failure or sudden cardiac death.

Validation of any identified association in two independent cohorts is a particular advantage of the present disclosure and a critical strength of the clinical study. Furthermore, verification in different populations and with and without secondary diagnoses, such as diabetes and hypercholesterolemia, represent another advantage of the disclosure. Several studies have reported an association between diabetes mellitus and increased urinary oxalate excretion or calcium oxalate deposition in renal allografts. The present disclosure is the first to show an association with cardiovascular disease and cardiac death and providing means to prevent or to treat said disease.

In some embodiments, oxalate-related cardiovascular disease is associated with calcium oxalate deposition in organs in a non-PH patient receiving dialysis treatment.

Oxalates are the salts and esters of oxalic acid. Insufficient or failed renal function leads to progressive hyperoxalemia, an increase in the content of oxalic acid in the blood plasma, and supersaturation of plasma calcium oxalate. The concentration of oxalate in plasma or serum is critical and increased oxalate concentrations at supersaturation level can lead to the formation of calcium oxalate crystals in the distal tubules and the connecting duct with subsequent formation of calcium oxalate depositions or calcification of tissues and organs, e.g. heart, bones, soft tissue, kidneys, skin, vessels including arteries and veins. Oxalate crystal-related inflammation may occur.

As evident in Example 1 and 2, non-PH dialysis patients in both cohorts exceeded the threshold of 30 µM for supersaturation of calcium oxalate, wherein supersaturation of calcium oxalate leads to formation of calcium-oxalate microcrystals and oxalate deposition in organs, and vascular and cardiac damage. The inventors provide a pathophysiological link between oxalate, compromised cardiac function, and sudden cardiac death. The excess mortality risk in both cohorts is unexpectedly significantly evident in patients presenting supersaturation of calcium oxalate levels. In one embodiment, the invention provides measures to prevent, to stop or to decrease formation of oxalate and calcium oxalate deposits at therapeutically relevant levels. Already in early stages of oxalate related cardiovascular disorder, calcification and cardiac events become visible, such as heart failure, as evident in the Examples 3 and 4.

In one embodiment, the composition comprising Lumasiran and/or Nedosiran prevents, diminishes or stops oxalate-related disorders, calcium oxalate deposition, and calcium oxalate deposition related disorders from progression or occurring. A composition according to the present disclosure is further capable of reducing or eliminating oxalate formation, thereby reducing systemic oxalate deposition.

In one embodiment, the invention also provided herein are novel medicinal uses of a composition comprising Lumasiran and/or Nedosiran in view of the improved effects shown herein.

In some embodiments, oxalate-related cardiovascular disease is associated with plasma or serum oxalate concentrations >20µM, preferably >30µM, in a non-PH patient receiving dialysis treatment.

Oxalate-related disorders progress with sustained increase of the ratio of total oxalate to free oxalate. As evident in the examples 2-4, non-PH patients receiving dialysis with oxalate concentrations ≥30 µM have a 2.9-fold higher mortality as patients below this cut-off (Figure 2A). Non-PH patients receiving dialysis with oxalate concentrations ≥40 µM have a more than 4-fold increased mortality compared to patients with oxalate concentrations <40 µM (Figure 2B). The inventors disclose evidence of an association between oxalate concentrations in serum or plasma and excess mortality in non-PH patients receiving dialysis.

In some embodiments, oxalate concentrations are associated with cardiovascular events, preferably sudden cardiac death and congestive heart failure in a non-PH patient receiving dialysis treatment.

In one embodiment, the composition comprising Lumasiran and/or Nedosiran is intended for use in the treatment and/or prevention of a calcium-oxalate deposition related cardiovascular disorder, wherein said use comprise the treatment and/or prevention of cardiovascular events and cardiovascular mortality, preferably sudden cardiac death, cardiac arrhythmias or congestive heart failure. In one embodiment, the composition comprising Lumasiran and/or Nedosiran is intended for use in the treatment and/or prevention of a calcium-oxalate deposition related mortality.

Putative causes and strategies to elucidate the pathophysiology of sudden cardiac death in dialysis patients have been extensively studied in the art, and a guidance for dialysis prescription exist, comprising known factors such as electrolyte shifts, acid-base balance, volume control, blood pressure fluctuations and other important dialysis parameters, but without succeed. The inventors surprisingly identified oxalate as a significant target.

In some embodiments, said composition reduces hepatic oxalate production and thus lowers plasma or serum oxalate concentrations in a non-PH patient receiving dialysis treatment.

The major enzymes involved in endogenous hepatic oxalate synthesis include glycolate oxidase (GO), alanine:glyoxylate aminotransferase, D-amino acid oxidase, and lactate dehydrogenase (LDH). In hepatic oxalate metabolism, glycolate is oxidized to glyoxylate, followed by conversion of glyoxylate to oxalate. The alanine:glyoxylate aminotransferase catalyzes the breakdown of glyoxylate. The conversion of glyoxylate to oxalate can be catalyzed by GO or LDH. In one embodiment, the combined clinical trial of two independent, complementary studies identifies oxalate concentration as a risk factor for mortality and especially sudden cardiac death in non-PH dialysis patients.

In the state of the art, measures are known to decrease oxalate intake by appropriate dietary changes. For the reduction of endogenous oxalate production, preferably hepatic oxalate production, RNAi therapeutics have been developed, i.e. Lumasiran and Nedosiran, to block mRNA translation of proteins, i.e. GO and LDH, involved in the breakdown of endogenous glyoxylate to oxalate. In the prior art, only the use of Lumasiran and Ledosiran in PH 1-3 patients has been disclosed and clinically tested.

In one embodiment, Lumasiran or Nedosiran reduces hepatic oxalate production in a non-PH patient receiving dialysis treatment and thus lowers plasma or serum oxalate concentrations at therapeutically relevant levels wherein said reduced hepatic oxalate production is mediated by Lumasiran blocking GO mRNA translation or Nedosiran blocking LDH mRNA translation.

In one embodiment, the combined administration of Lumasiran and Nedosiran provides an improved effect to reduce the hepatic oxalate production when administered to a non-PH patient receiving dialysis treatment.

In some embodiments, potency of Lumasiran or Nedosiran of the invention is determined in reference to the number of copies of a dsRNA present in the cytoplasm of a target cell that are required to achieve a certain level of target gene knockdown. Preferably, Lumasiran or Nedosiran are potent if one is capable of generating of at least 50% knockdown of a target mRNA when present in the cytoplasm of a target cell compared to an untreated target cell. In one embodiment, the efficacy of Lumasiran and Nedosiran to target genes can be obtained by quantification of mRNA levels of said target genes, preferably by quantitative PCR.

In some embodiments, the target cell for Lumasiran and Nedosiran is a hepatic cell.

In one embodiment, the present disclosure provides measures and a therapeutic strategy comprising Lumasiran and/or Nedosiran for the treatment of non-PH dialysis patients to reduce endogenous oxalate production at therapeutically relevant levels. In one embodiment, said measures and a therapeutic strategy includes the composition comprising Lumasiran and/or Nedosiran provided herein, wherein said composition can be administered additionally to dietary measures or other treatments or as independent therapeutic for the reduction of plasma and serum oxalate concentrations at therapeutically relevant levels.

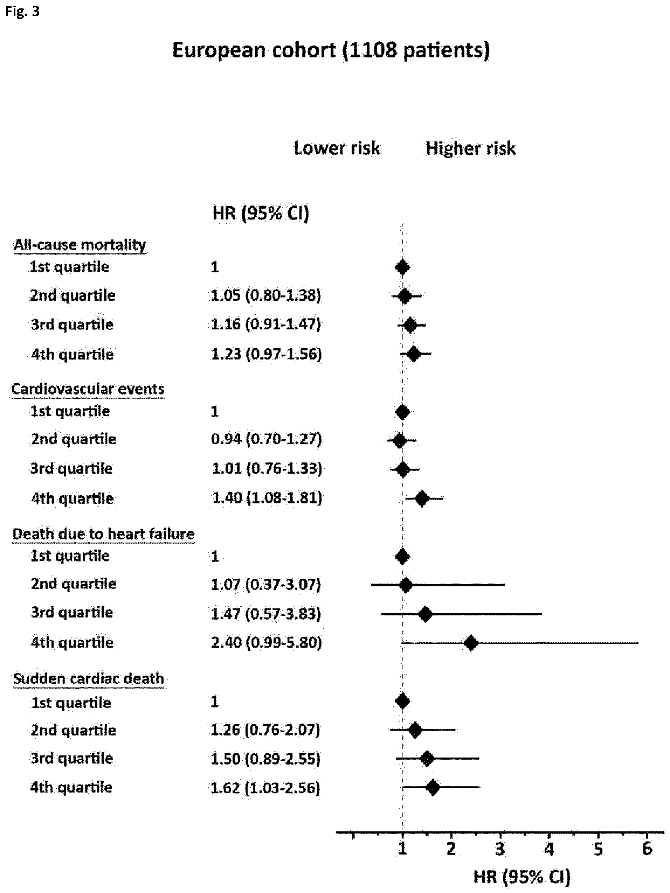

As evident in Example 4, the incidence of combined cardiovascular events, as presented for the 4D Study, is markedly increased at higher oxalate concentrations (Figure 3). Non-PH dialysis patients in the highest oxalate quartile surprisingly had a 40% increased risk to reach the combined endpoint. A skilled person could not have been expecting that higher baseline oxalate concentrations are also associated with incrementally higher risk of death due to congestive heart failure. The patients in the highest quartile are unexpectedly more than 2-fold more likely to die of heart failure than patients in the lowest quartile (Table 5 and Figure 3). Furthermore, the inventors disclose the risk of sudden cardiac death as a separate outcome measure increasing incrementally and significantly with higher oxalate quartiles. Patients in the highest quartile exhibit a 62% higher sudden death risk compared to those in the lowest quartile, as evident in Figure 3 and Example 4.

As skilled person could not expect that the associations between plasma and serum oxalate and myocardial infarction as well as stroke were also consistent with the combined events, heart failure and sudden cardiac death. These are a highly relevant findings, opening up for improved treatments or preventions of oxalate-related cardiovascular disorders including those associated with calcium oxalate deposition, preferably sudden cardiac death, heart failure and excess mortality by using a composition according to the present disclosure.

This also evidences measures to treat oxalate concentrations in plasma and serum in non-PH dialysis patients and the high demand for an appropriate means, as provided in this disclosure.

In one embodiment, said measures and therapeutic strategy are suitable to prevent excess mortality and cardiac fatal disorders. In another embodiment, said measures and therapeutic strategy are suitable to treat and to improve cardiovascular events associated with oxalate in plasma and serum and/or oxalate deposits in tissue and organs.

In some embodiments, said composition is administered subcutaneously, intravenously or orally at therapeutically relevant dosage at the time of dialysis treatment.

In one embodiment, the present disclosure also provides a method of treating and/or preventing an oxalate-related cardiovascular disorder as described herein, the method comprising administering to a subject in need thereof a pharmaceutically effective amount of a pharmaceutical composition as defined herein comprising a pharmaceutically effective amount of a pharmaceutical composition. In one embodiment, the Lumasiran or Nedosiran or their derivatives thereof are a pharmaceutically acceptable compound.

A preferred therapeutically relevant dosage has an inhibitory effect on hepatic endogenous oxalate production reducing oxalate concentration in serum or plasma, wherein any dosage is also administered before, during, at the end or after dialysis treatment.

The plasma oxalate concentrations in normal subjects ranges from 1.3-3.1 µM. Accordingly, in some embodiments, administration of the composition disclosed herein are useful for reducing plasma or serum oxalate concentrations such that a subject no longer exhibits levels of serum or plasma oxalate above normal ranges or associated with clinical hyperoxalemia. In some embodiments, administration of the composition disclosed herein reduces a subject's serum or plasma oxalate to <30µM after treatment.

In some embodiments, treatment or prevention is accomplished with a single dose, e.g., one-time treatment, of composition described herein. In some embodiments, the single dose achieves durable treatment or prevention. Durable treatment and/or prevention, as used herein, includes treatment and/or prevention lasting at least from one dialysis treatment to the next following dialysis treatment or every 2,3,4,5,6,7,8,9,or 10 dialysis treatments or for the duration of the patient's life. In some embodiments, treatment or prevention is accomplished with more than one dose, e.g., two-time treatment, three-time treatment, or for the duration of the patient's life comprising a dosage regiment described herein, of the composition described herein. Treatment or prevention, as used herein, at the time of dialysis treatment is most advantageous for efficacy, gentle on the patient, and is also associated with less side effects.

In one embodiment, the treatment or prevention in non-PH1 patients receiving continuous dialysis treatment is performed during dialysis treatment with a time interval and dosing schedule as described herein. In one embodiment, treatment or prevention in non-PH1 patients receiving intermittent dialysis treatment is preferably administered at a time of dialysis treatment. In one embodiment, the durable treatment or prevention also comprises one or more treatments and/or prevention in a period between one dialysis treatment and another, next dialysis treatment, wherein one or more further dialysis treatments can be performed in said period. In one embodiment, the temporary treatment or prevention also comprises one or more treatments and/or prevention in a period between one dialysis treatment and another, next dialysis treatment, wherein one or more further dialysis treatments can be performed in said period.

In one embodiment, the number of said treatments or preventions in a period between one dialysis treatment and the subsequent next dialysis treatment can also vary to each subsequent period between two or more consecutive dialysis treatments. In some embodiments, a time interval between treatment or prevention comprises a period of several days (2, 3, 4, 5, 6, or 7), several weeks (1, 2, 3, 4, 5, 6, 7, or 8), or months (2, 3, 4, 5, or 6), preferably 4 weeks to 3 months. In some embodiments, the time interval from one treatment or prevention to the next subsequent treatment can be the same, nearly the same or can change.

In some embodiments, the invention comprises a method of treating or preventing an oxalate-related cardiovascular disorder in subject, wherein said method comprises administering one or more of the compositions or pharmaceutical formulations described herein. In one embodiment, the compositions and/or formulations are administered subcutaneously, intravenously or orally.

Another major advantage of treatment as described here is the minimum incidence of side effects, if any.

In some embodiments, Lumasiran and/or Nedosiran are administered in concentrations or amounts sufficient to provide a therapeutic effect. A skilled person can determine such an effect without undue effort. This amount relates to a therapeutically effective amount when a compound is used alone, or to a therapeutically effective amount when a compound is used in combination with a second compound. In preferred embodiments, the compounds are administered in concentrations or amounts, or according to dosage regimes, already established in the art, such as those for which regulatory approval has been issued or granted (e.g. by the FDA or EMA), or in doses currently being assessed during phase 2 or 3 clinical trials, and/or according to the maximum allowed dose according to a phase I trial. In one embodiments, the compounds are administered in concentrations or amounts, lower than dosage regimes, already established in the art, such as those for which regulatory approval has been issued or granted (e.g. by the FDA or EMA), or lower than doses currently being assessed during phase 2 or 3 clinical trials, and/or lower than the maximum allowed dose according to a phase I trial.

In such a treatment, Lumasiran and/or Nedosiran of the composition may be administered simultaneously or sequentially to said patient, Lumasiran being administered before or after Nedosiran. Lumasiran and Nedosiran may also be administered by the same or a different administration route.

The above embodiments, regarding particular dosage, time and application type, are based on clinically allowed or currently trialed doses of the compounds described herein, being administered alone or combined as described herein.

In some embodiments, administered dosage and frequency is suitable to achieve a plasma or serum oxalate concentration <30µM and/or lower as before treatment initiation, preferably a plasma or serum oxalate concentration at least 20% lower as before treatment initiation.

Lumasiran and Nedosiran and derivatives thereof are potent and low toxicity compounds reducing or blocking hepatic oxalate production in non-PH dialysis patients. This combined beneficial effect could not be derived from any suggestion in the art.

In embodiments of the invention, the composition comprising Lumasiran and/or Nedosiran as described herein may be administered to any non-PH patient receiving dialysis, for example therapeutically (e.g. to patients with plasma or serum oxalate concentrations >20µM, preferably >30µM and/or cardiovascular disorder) or prophylactically (e.g. to patients who are at risk of having oxalate-related cardiovascular disease, or to patients with plasma or serum oxalate concentrations >20µM but have not yet developed serious health issues).

A skilled person is capable, based on the experimental and written support provided herein, to carry out the invention, in combination with common knowledge in the art. By employing the quantitative measures described in the examples, or by using alternative quantitative means for determining reductions in oxalate concentrations in plasma or serum, oxalate deposits, vascular calcification or cardiovascular events, the combination of pharmaceutical compounds and their necessary doses can be determined and adjusted in order to obtain beneficial effects due to the inventive application of the composition.

Preferred therapeutically relevant dosage has an inhibitory effect on hepatic endogenous oxalate production reducing oxalate concentration in serum or plasma, wherein said dosage reduces said oxalate concentration <30µM and/or lower as before treatment initiation, preferably a plasma or serum oxalate concentration at least 20% lower as before treatment initiation.

In one embodiment, composition described herein is administered to a non-PH dialysis patient with plasma or serum oxalate concentration >20µM, preferably >25µM, more preferably >30µM and with or without oxalate related cardiovascular diseases.

In one embodiment, the composition described herein is administered to a non-PH dialysis patient at risk having congestive heart failure, stroke and/or myocardial infarction.

In one embodiment, composition described herein is administered to a non-PH dialysis patient at risk to sudden cardiac death or overall excess mortality.

In one embodiment, the composition described herein is administered to a non-PH dialysis patient that suffers from oxalate deposits in organs or tissues, preferably calcium oxalate deposits in organs or tissues, more calcium oxalate deposits in vessels and/or the heart.

In some embodiments, administration of said composition prevents or reduces vascular calcification at therapeutically relevant levels caused by calcium oxalate.

The present invention also provides the composition used in the prevention or treatment of vascular calcification at therapeutically relevant levels caused by calcium oxalate in a subject.

In one embodiment, administration of said composition lowers oxalate concentration in plasma or serum, and reduces the risk of precipitation of calcium oxalate as a major component of coronary artery calcification in non-PH patients receiving dialysis treatment, thus preventing the initiation or progression of a cardiovascular disease.

In one embodiment, composition described herein is administered to a non-PH dialysis patient with oxalate-related vascular calcification.

In one embodiment, plasma or serum oxalate concentrations as therapeutic targets are determined following immediate cooling, deproteinization and acidification, and using an oxalate-oxidase assay, preferably as described for the Examples. Additionally, the invention includes a method of monitoring, determining or quantifying whether the composition comprising Lumasiran and/or Nedosiran reduces oxalate concentration in plasma or serum compared to oxalate concentration before treatment started, preferably as described for the Examples. In one embodiment, non-PH1 patients receiving intermittent dialysis undergo blood sampling directly before starting dialysis therapy, wherein said blood sampling can be used for quantifying plasma or serum oxalate concentrations as therapeutic targets.

The invention further relates to a composition comprising the nucleic acid, dsNA, or hybridization complex of Lumasiran and/or Nedosiran or its salts thereof, wherein said nucleic acid, dsNA, or hybridization complex is present in an amount effective to reduce cytosolic mRNA levels of its target genes when introduced into a cell of a non-PH patient receiving dialysis treatment.

In one embodiment, Lumasiran and/or Nedosiran can be chemically modified.

In some embodiments, nucleic acid, dsNA, or hybridization complex is preferably coated to a pharmaceutically acceptable carrier and formulated in a pharmaceutically buffered solution. In one embodiment, the pharmaceutical combination as described herein is characterized in that the Lumasiran and its derivatives thereof is in a pharmaceutical composition in admixture with a pharmaceutically acceptable carrier, and Nedosiran and its derivatives thereof is in a separate pharmaceutical composition in admixture with a pharmaceutically acceptable carrier. The pharmaceutical combination of the present invention can therefore in some embodiments relate to the presence of two separate compositions or dosage forms in proximity to each other. The compounds in combination are not required to be present in a single composition.

Single preparations, combinational preparations or compositions are known to the skilled person who is able to evaluate compatible carrier materials and formulation forms suitable for both active compounds in the combination. In some embodiments, the quantity of active ingredient that can be combined with the carrier materials to produce a single dosage varies depending on the patient being treated and the particular route of administration.

The invention further relates to a method for preparing the composition comprising Lumasiran and/or Nedosiran according to claim 10 and 11.

In one embodiment, for preparing the composition comprising Lumasiran and/or Nedosiran of the invention, said compounds are added directly or may be complexed with lipids (e.g., cationic lipids), packaged in liposomes, or otherwise delivered to target cells or tissues. The nucleic acid or nucleic acid complexes may be delivered locally to relevant tissues ex vivo or in vivo by direct dermal application, transdermal application, or injection, with or without their incorporation into biopolymers.

The invention further relates to a kit comprising Lumasiran and/or Nedosiran according to any of the preceding claims, and instructions for its use. In one embodiment, Lumasiran is in a pharmaceutical composition in admixture with a pharmaceutically acceptable carrier, and Nedosiran is in a separate pharmaceutical composition in admixture with a pharmaceutically acceptable carrier. In one embodiment, Lumasiran and Nedosiran are combined in a single pharmaceutical composition in admixture with a pharmaceutically acceptable carrier. In one embodiment, Lumasiran and Nedosiran are present in a kit, in spatial proximity but in separate containers and/or compositions.

The invention further relates to the pharmaceutical combination for use in the treatment and/or prevention of an oxalate related cardiovascular disorder in a subject and/or a medical condition associated with serum or plasma oxalate concentrations at therapeutically relevant levels and/or oxalate deposits.

Further examples of pharmaceutical compositions, treatments, patients, target genes, and targets are described in detail below.

The features of the invention regarding methods of treatment and diagnosis, and descriptions of the composition comprising Lumasiran and/or Nedosiran for use in the treatment of various medical conditions, apply to the composition itself, and vice versa.

### DETAILED DESCRIPTION OF THE INVENTION

The present invention relates to the treatment or prevention of an oxalate-related disorder in a non Primary Hyperoxaluria patient receiving dialysis.

All words and terms used herein shall have the same meaning commonly given to them by the person skilled in the art, unless the context indicates a different meaning. All terms used in the singular shall include the plural of that term and vice versa.

In accordance with the present disclosure, there is provided herein a composition comprising lumasiran and/or nedosiran, and in the form of a medicament, having an effect on oxalate-related disorders and in particular calcium oxalate deposition-related disorders, such as calcium oxalate deposition-related disorders involving hyperoxalemia. Hyperoxalemia is characterized by high oxalate concentrations in plasma and serum, leading, for example, to the formation and deposition of calcium oxalate complexes in the body. It has proven difficult in the art to identify pharmaceutical compositions that have an effect on the calcium oxalate deposits formed per se and/or in hyperoxalemia. If such an effect is possible, it may help delay or stop the formation of the oxalate deposits or help dissolve the oxalate deposits and possibly reverse an associated disorder, such as a cardiovascular complication. Such an effect is found herein for a composition according to the disclosure, which is further described below.

### Lumasiran and Nedosiran

Lumasiran developed by Alnylam is an investigational, subcutaneously administered RNA interference (RNAi) therapeutic targeting hydroxyacid oxidase 1 (HAO1) being developed for the treatment of primary hyperoxaluria type 1 (PH1) (patents US202002062581; US20200165616). HAO1 encodes glycolate oxidase (GO). By silencing HAO1 and degrading the GO enzyme, Lumasiran inhibits oxalate production - the metabolite that directly affects the pathophysiology of PH1. PH1 is an extremely rare disease characterized by excessive oxalate formation. Elevated urinary oxalate levels have been associated with disease progression to end-stage renal failure and other systemic complications of the disease (https://www.ncbi.nlm.nih.gov/books/NBK1283).

Lumasiran met the primary endpoint of the ILLUMINATE-A study (NCT03681184)) with a 53.5% mean reduction in urinary oxalate compared to placebo (p = 1.7 x10-14). Furthermore, urinary oxalate concentration also decreased by 65.4% from baseline. All secondary endpoints assessed in the study were met. This included the proportion of patients with near normalization (84%) or normalization (52%) of urinary oxalate levels compared to 0% with placebo. Lumasiran administration showed a promising safety and tolerability profile with no severe adverse events. Mild injection site reactions were the most common drug-related adverse events. Lumasiran features Alnylam's Enhanced Stabilization Chemistry (ESC) GalNAc conjugate technology, which allows subcutaneous dosing with enhanced efficacy and prolonged duration of action and broad therapeutic range. Based on the results of the ILLUMINATE-A study, Alnylam submitted a Marketing Authorization Application (MAA) for lumasiran to the European Medicines Agency (EMA). This application has been cleared by the EMA for the Accelerated Assessment process. Lumasiran received orphan drug designation in both the EU and the U.S., was granted Priority Medicines (PRIME) designation by the European Medicines Agency (EMA). In the meantime, Lumasiran has been officially approved by the EMA and the FDA since the end of 2020.

Nedosiran developed by Dicerna Pharmaceuticals is an investigational, subcutaneously administered RNA interference (RNAi) therapeutic targeting the hepatic lactate dehydrogenase enzyme, an enzyme that catalyzes the final step in the glyoxalate metabolism pathway that can lead to oxalate overproduction in patients with PH1, PH2 or PH3 (WO2015100436). Nedosiran uses a GalXC technology from Dicerna Pharamceuticals. The LDH enzyme inhibition occurs specifically in the liver due to the incorporation of GalNAc targeting ligands in nedosiran that bind specifically to the Asialoglycoprotein receptors (ASGPR) on hepatic cell surfaces. Nedosiran is the RNAi therapy in development for the treatment of all three known types of PH. The PHYOX2/3 multicenter pivotal trial (NCT03847909) finished enrollment in December 2020. An October 2020 interim analysis of the PHYOX3 study showed that all participants receiving nedosiran, regardless of PH1 or PH2 subtype, achieved normalization or near normalization of urinary oxalate levels by day 180. Of the 13 participants who reached day 180 at the time of the interim analysis, 10 (100%) of the participants with PH1 and two of the three participants (67%) with PH2 achieved normal urinary oxalate excretion.

As used herein, the terms "oxalate crystal deposits", "oxalate deposits", "deposits" are interchangeable with, or at least related to, the terms "calcium oxalate deposits" and "calcium oxalate complexes", which are also used herein..

### RNA-interference

RNA-interference (RNAi) is a natural cellular process for regulation to silencing of genes. As used herein, the term "interfering RNA" or "RNAi," "dsNA," or "interfering RNA sequence" includes single-stranded RNA (e.g., mature miRNA, ssRNAi oligonucleotides, ssDNAi oligonucleotides), double-stranded RNA (ie, duplex RNA such as siRNA, dicer substrate dsRNA, shRNA, aiRNA, or pre-miRNA), or hybridization complexes, i.e. a DNA-RNA hybrid or a DNA-DNA hybrid, capable of reducing or inhibiting expression of a target gene or sequence (e.g. by mediating the degradation or inhibition of translation of mRNAs complementary to the interfering RNA sequence) when the interfering RNA is in the same cell as the mRNA of the target gene or sequence. Interfering RNA thus refers to single-stranded RNA that is complementary to a target mRNA sequence or to double-stranded RNA formed by two complementary strands or by a single, self-complementary strand. The interfering RNA may have substantial or complete identity to the target gene or sequence. The sequence of the interfering RNA may correspond to the full-length target gene or a partial sequence thereof. Preferably, the interfering RNA or DNA/RNA molecules are chemically synthesized. The disclosures of each of the above patent documents for Lumasiran and Nedosiran are incorporated herein by reference in their entirety for all purposes.

The term "inhibition of target gene expression" refers to the ability of an interfering RNA (i.e. Lumasiran and/or Nedosiran) to silence, reduce, or inhibit the expression of a target gene at mRNA level. To examine the extent of gene silencing, a test sample (e.g., a sample of cells in culture that express the target gene) or a test mammal (e.g., a mammal such as a human or an animal model such as a rodent (e.g., a mouse) or a non-human primate model (e.g., a monkey)) is exposed to an interfering RNA (i.e. Lumasiran and/or Nedosiran) that silences, reduces, or inhibits the expression of the target gene. The expression of the target gene in the test sample or test mammal is compared to the expression of the target gene in a control sample (e.g., a sample of target cells in culture that express the target gene) or a control mammal (e.g., a mammal such as a human or an animal model such as a rodent (e.g., mouse) or a non-human primate model (e.g., monkey)) that is not exposed to or administered the interfering RNA, i.e. Lumasiran and/or Nedosiran. A value of 100% may be assigned to the expression of the target gene in a control sample or control mammal. In particular embodiments, silencing, inhibition, or reduction of expression of a target gene is achieved when the level of target gene expression in the test sample or test mammal relative to the level of target gene expression in the control sample or control mammal is about 95%, 90%, 85%, 80%, 75%, 70%, 65%, 60%, 55%, 50%, 45%, 40%, 35%, 30%, 25%, 20%, 15%, 10%, 5%, or 0%. In other words, the interfering RNA (i.e. Lumasiran and/or Nedosiran) silences, reduces, or inhibits expression of a target gene by at least about 5%, 10%, 15%, 20%, 25%, 30%, 35%, 40%, 45%, 50%, 55%, 60%, 65%, 70%, 75%, 80%, 85%, 90%, 95%, or 100% in a test sample or test mammal compared to the level of target gene expression in a control sample or control mammal that has not been exposed to or administered the interfering RNA, i.e. Lumasiran and/or Nedosiran. Suitable assays for determining the level of target gene expression include, without limitation, assaying protein or mRNA levels using techniques known to those skilled in the art, such as dot blots, Northern blots, in situ hybridization, ELISA, immunoprecipitation, enzyme function, and phenotypic assays known to those skilled in the art.

As used herein, the term "target gene" refers to a nucleic acid sequence coding for a protein involved in hepatic endogenous oxalate production, preferably for glycolate oxidase (GO), alanine:glyoxylate aminotransferase, D-amino acid oxidase, and lactate dehydrogenase (LDH), more preferably for GO or LDH

As used herein, the term "target cell" refer to a living cell in which a target gene for an interfering RNA targeted for oxalate therapy, i.e. Lumasiran and/or Nedosiran, is expressed.

The term "nucleic acid" as used herein refers to a polymer containing at least two deoxyribonucleotides or ribonucleotides in single- or double-stranded form, and includes DNA, RNA, and hybrids thereof. DNA may be in the form of, for example, antisense molecules, RNA-DNA duplexes, a PCR product, chimeric sequences, derivatives, and combinations of these groups. RNA can be in the form of small interfering RNA (siRNA), dicer substrate dsRNA, small hairpin RNA (shRNA), asymmetric interfering RNA (aiRNA), microRNA (miRNA), mRNA and combinations thereof. DNA and RNA can form DNA-RNA hybrids.

### Therapeutically effective and therapeutically relevant

The phrase "therapeutically effective" is intended to include, within the scope of sound medical judgment, excessive toxicity, irritation, allergic reactions, and/or other problems or complications, but commensurate with a reasonable benefit/risk ratio. As used herein to refer to the compounds, compositions, combinations and/or dosage forms suitable for use in contact with a subject that produces a result that in and of itself helps to treat and/or cure a disease.

A "therapeutically relevant amount", "therapeutically relevant dosage" or "therapeutically effective amount" of an agent or therapeutic means, such as an interfering RNA i.e. Lumasiran and/or Nedosiran, is an amount sufficient to produce the desired effect, e.g., inhibition of expression of a target sequence relative to the normal level of expression detected in the absence of an interfering RNA. Inhibition of expression of a target gene or sequence is achieved when the level obtained with an interfering RNA relative to the control is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring expression of a target gene or sequence include, for example, assaying protein or RNA levels using techniques known to those skilled in the art, such as quantitative PCR, Northern blots, Western blots, in situ hybridization, ELISA, enzyme function, and phenotypic assays known to those skilled in the art.

By "decrease," "decreasing," "reducing," "lower" or "diminishing" of oxalate concentration by an interfering RNA, i.e. Lumasiran and/or Nedosiran, is meant as a detectable drop in oxalate production to a given interfering RNA, i.e. Lumasiran and/or Nedosiran. The extent of decrease in in oxalate concentration by an interfering RNA, i.e. Lumasiran and/or Nedosiran, may be determined relative to the level of oxalate concentration without the presence of an interfering RNA, i.e. Lumasiran and/or Nedosiran, preferably before initiation of treatment with Lumasiran and/or Nedosiran. A detectable decrease may be about 5%, 10%, 15%, 20%, 25%, 30%, 35%, 40%, 45%, 50%, 55%, 60%, 65%, 70%, 75%, 80%, 85%, 90%, 95%, or 100% than the oxalate concentration detected before initiation of treatment with Lumasiran and/or Nedosiran. A decrease in oxalate concentration by the interfering RNA is typically understood and measured by a decrease in oxalate production by a responder cell in vivo or a decrease in oxalate concentration in plasma and serum of a dialysis patient after first and/or any further administration of the interfering RNA, i.e. Lumasiran and/or Nedosiran.

"Systemic administration," as used herein, refers to the administration of a composition that results in broad biodistribution of Lumasiran and/or Nedosiran within an organism. Systemic administration means exposing a therapeutic amount of an agent to preferred parts of the body. Systemic administration of the composition may be accomplished by any means known in the art, e.g., intravenously, subcutaneously, intraperitoneally,

### Oxalate, oxalate deposits, and oxalate-related disorders

The terms "oxalate crystal deposits", "oxalate deposits", "deposits" as used herein, or the like are interchangeable with, or at least related to, the terms "calcium oxalate deposits" and "calcium oxalate complexes" also used herein. Oxalate deposition in the body is sometimes referred to as "oxalosis". Oxalate can be found circulating in plasma and serum as free oxalate, free oxalate bound to divalent metals, protein- and lipid-associated oxalate, and solid crystals of oxalate of divalent metals. Oxalate-related disorders progress with sustained increases in the ratio of total oxalate to free oxalate. Hyperoxalemia determines the clinical increase of oxalic acid levels in the blood plasma. For methodological reasons, oxalate is usually determined in plasma. Oxalates are the salts and esters of oxalic acid.

Use of the pharmaceutical composition is particularly contemplated in the treatment of a condition associated with elevated plasma and serum oxalate levels, frequent systemic oxalate crystal deposition, and impaired renal function. Since excretion of dissolved oxalate deposits may take some time after treatment with Lumasiran and/or Nedosiran, treatment periods of several months to several years are envisaged for individuals suffering from such disorders. Calcification and oxalate deposits of soft tissue is a common problem in dialysis receiving patients.

Lumasiran and nedosiran mediate the active reduction of the formation of oxalate by inhibiting expression of target genes involved in oxalate production pathway. Such a reduction in oxalate levels may facilitate the transfer of existing free oxalate from plasma/serum to the intestine. This process disturbs the balance between free plasma oxalate, protein-associated oxalate, and deposited calcium oxalate. The disruption of the equilibrium can drive free oxalate and lead to dissolution of deposited calcium oxalate crystals. Since crystal dissolution is controlled by the surrounding mass concentration of the counterion in question, crystals may briefly dissolve below the saturation concentration. As a result of dissolution, increased oxalate in plasma and urine may also occur intermediately. This encompassed by the present invention.

Methods for quantifying plasma or serum oxalate concentrations as therapeutic targets comprise immediate cooling, deproteinization and acidification, and use of an oxalate-oxidase assay. In one embodiment, non-PH1 patients receiving intermittent dialysis undergo blood sampling directly before starting dialysis therapy, wherein said blood sampling can be used for quantifying plasma or serum oxalate concentrations as therapeutic targets. The methods are described in the Examples in more detail.

### Dialysis

The term dialysis, as used herein, refers to all forms of dialysis, including hemodialysis, hemofiltration, hemodiafiltration, hemoperfusion, and peritoneal dialysis, preferably hemodialysis and peritoneal dialysis. In medicine, dialysis comprises therapy methods that serve to remove urinary substances from the blood (kidney replacement procedures). Kidney failure is a serious complication that requires independent treatment. Dialysis can control kidney failure and is therefore an important and often the only treatment, but tends to be insufficient to dispose of excess oxalate, so that deposits of oxalate can occur in the kidney and other organs and tissues.

Hemodialysis and peritoneal dialysis are preferred types of dialysis therapies. Hemodialysis treatment uses the patient's blood to remove waste, toxins and excess water from the patient. The patient is connected to a hemodialysis machine and the patient's blood is pumped through the machine. Catheters are inserted into the patient's veins and arteries to allow blood to flow to and from the hemodialysis machine. The blood flows through a machine dialyzer, which removes waste, toxins and excess water from the blood. The cleansed blood is returned to the patient. Hemodialysis treatment lasts several hours and is performed in a treatment center.

In peritoneal dialysis, a dialysis solution, also called dialysate, is infused into the patient's peritoneal cavity through a catheter. The dialysate comes into contact with the peritoneal membrane of the abdominal cavity. Waste products, toxins and excess water pass from the patient's bloodstream through the peritoneal membrane into the dialysis fluid due to diffusion and osmosis, i.e., an osmotic gradient is created across the membrane. The spent dialysate is drained from the patient, removing waste, toxins and excess water from the patient. Peritoneal dialysis therapies also include continuous ambulatory peritoneal dialysis ("CAPD"), automated peritoneal dialysis ("APD"), tidal flow APD, and continuous flow peritoneal dialysis ("CFPD").

The dialysis treatment methods can be used and implemented in various hemodialysis and peritoneal dialysis technologies, as described in, for example, U.S. Patent Nos. 5,244,568 , 5,247,434 , 5,350,357 , 5,662,806 , 6,592,542 , and 7,318,892 .

### Cardiovascular disease

Cardiovascular disease is a large class of diseases that affect the heart or blood vessels (arteries and veins). Cardiovascular diseases include arrhythmias, vascular disease, myocardial infarction, heart failure, myocarditis, atherosclerosis, restenosis, coronary heart disease, coronary artery disease, atherosclerotic cardiovascular disease, arterial hypertension, cardiac fibrosis, stroke, sudden cardiac death syndrome, heart failure, ischemic cardiomyopathy, myocardial infarction, coronary artery calcification. These diseases have similar causes, mechanisms, and treatments. Most cardiovascular diseases have common risk factors, including inflammation, fibrosis, diabetes, cholesterol, and vascular deposits. Oxalate deposition in the heart, termed as "Cardiac oxalosis", may lead to congestive heart failure, compromised cardiac function, cardio-embolism, vascular and cardiac damage, diastolic dysfunction, and cause conduction disturbances and sudden death.

### Composition

For purposes of the present invention, a composition as used herein is also a "pharmaceutical composition" and combines the presence of one or more RNAi therapeutic, i.e. Lumasiran and/or Nedosiran, preferably in close proximity to each other. In one embodiment, the combination is suitable for combined administration.

In one embodiment, the pharmaceutical composition described herein is characterized in that the Lumasiran is present in admixture with a pharmaceutically acceptable carrier and the Nedosiran is present in a separate pharmaceutical composition in admixture with a pharmaceutically acceptable carrier. Thus, in some embodiments, the pharmaceutical combination of the present invention may refer to the presence of two separate compositions or dosage forms in close proximity to each other. The active ingredients in combination are not required to be present in a single composition.

In one embodiment, the pharmaceutical combination described herein is characterized in that the Lumasiran and/or Nedosiran according to any one of the preceding claims are present in a kit, in close proximity, but in separate containers and/or compositions. Producing a kit is within the skill of one skilled in the art. In one embodiment, separate compositions comprising two separate active ingredients may be packaged and marketed together as a combination. In other embodiments, offering the two active ingredients in combination, e.g., in a single catalog, but in separate packages is understood to be a combination.

In one embodiment, the pharmaceutical combination described herein is characterized in that Lumasiran and/or Nedosiran according to any one of the preceding claims are combined in a single pharmaceutical composition in admixture with a pharmaceutically acceptable carrier. Combination preparations or compositions are known to those skilled in the art who are able to evaluate compatible carrier materials and formulation forms suitable for both active ingredients in the combination.

### Inhibitors:

According to the present invention, an "inhibitor" in the context of "mRNA translation inhibitor" is considered to be any agent, substance, compound, molecule, or other means that results in slowing, repressing, blocking, or otherwise interfering with or negatively affecting the activity, function, expression, or signaling that said target induces, performs, or exhibits in the absence of the inhibitor. The terms "agent", "substance", "compound", "molecule" can be used interchangeably. For example, RNAi therapeutics, i.e. Lumasiran and/or Nedosiran, may effect mRNA translation of a target gene involved in oxalate production within a cell, either directly or indirectly. The inhibitors as described herein may also be termed "agents". References to the "agents" in the context of the combinations and methods described herein are to be understood as RNA interference and mRNA translation inhibitor. Preferred inhibitors are those described herein.

### Combined administration:

According to the present invention, the term "combined administration" and "combined therapy", otherwise known as co-administration or joint treatment, encompasses in some embodiments the administration of separate formulations of the compounds described herein, whereby treatment may occur within minutes of each other, in the same hour, on the same day, in the same week or in the same month as one another. Alternating administration of two agents is considered as one embodiment of combined administration. Staggered administration is encompassed by the term combined administration, whereby one agent or treatment may be administered, followed by the later administration of a second agent or treatment, optionally followed by administration of the first agent or treatment, again, and so forth. Simultaneous administration of multiple agents or treatments is considered as one embodiment of combined administration. Simultaneous administration encompasses in some embodiments, for example the taking of multiple compositions comprising the multiple agents or treatments at the same time, e.g. by infusions, injections, dialysis treatment or orally by ingesting separate tablets simultaneously, preferably any administration via dialysis treatment. A combination medicament, such as a single formulation comprising multiple agents or treatments disclosed herein, and optionally additional cardiovascular-managing medicaments, may also be used in order to co-administer the various components in a single administration or dosage.

A combined therapy or combined administration of one agent or treatment may precede or follow administration with another agent or treatment to be combined, by intervals ranging from minutes to weeks. In embodiments where the second agent or treatment and the first agent or treatment are administered separately, one would generally ensure that a significant period of time did not expire between the time of each delivery, such that the first and second agents or treatment would still be able to exert an advantageously combined effect on a treatment site. In such instances, it is contemplated that one would contact the subject with both modalities within about 1h to 4 weeks of each other. In some situations, it may be desirable to extend the time period for treatment significantly, however, where several days (2, 3, 4, 5, 6 or 7) to several weeks (1, 2, 3, 4, 5, 6, 7 or 8) or even months (2, 3, 4, 5 or 6) lapse between the respective administrations.

In the meaning of the invention, any form of administration of the multiple agents described herein is encompassed by combined administration, such that a beneficial additional therapeutic effect, preferably a synergistic effect, is achieved through the combined administration of the two agents.

### Medical Indications:

In one aspect of the present invention there is provided a pharmaceutical composition according to the invention as herein described for use in the treatment of a medical condition associated with oxalate levels in serum and plasma, wherein the medical condition associated with oxalate levels in serum and plasma is preferably a cardiovascular disease or a calcium oxalate deposition associated cardiovascular disease.

As used herein, the "patient" or "subject" refers to a human, preferably a patient receiving dialysis, but can also be any other mammal, such as a domestic animal (e.g. a dog, cat or the like), a farm animal (e.g. a cow, sheep, pig, horse or the like) or a laboratory animal (e.g. a monkey, rat, mouse, rabbit, guinea pig or the like). The term "patient" preferably refers to a "subject" suffering from or suspected of suffering from a cardiovascular disease and receiving a dialysis, excluding those suffering from Primary Hyperoxaluria 1-3 (PH).

Primary hyperoxaluria is a rare disorder of glyoxylate metabolism. It is characterized by excess oxalate. The resulting symptoms range from occasional kidney stones to recurrent nephrolithiasis and nephrocalcinosis to end-stage renal disease and systemic oxalosis. The disease is divided into three distinct types: Primary hyperoxaluria type 1, type 2, and type 3. The patient group receiving a dialysis treatment as described herein excludes patients diagnosed with Primary hyperoxaluria type 1, type 2, or type 3.

As used herein, a "non PH dialysis patient with symptoms of a cardiovascular disease" is a subject who presents with vascular calcification, myocardial infarction, congestive heart failure, survived cardiac arrest, arrhythmias, cardiovascular events, and has an increased risk of death. As used herein, the term "a non PH dialysis patient that is at risk of developing oxalate-related cardiovascular disorders" relates to a subject or patient, preferably distinct from any given person in the general population, who has an increased (e.g. above-average) risk of developing oxalate-related cardiovascular disorders and/or oxalate deposits related cardiovascular disorders. In some embodiments, said patient has symptoms of vascular calcification or symptoms of a myocardial infarction or symptoms of a congestive heart failure or symptoms of cardiovascular events or symptoms of an increased risk of death. In some embodiments, said patient has no symptoms of symptoms of vascular calcification or symptoms of a myocardial infarction or symptoms of a congestive heart failure or symptoms of cardiovascular events or symptoms of an increased risk of death. In some embodiments, the said patient at risk of developing symptoms of vascular calcification or symptoms of a myocardial infarction or symptoms of a congestive heart failure or symptoms of cardiovascular events or symptoms of an increased risk of death presents serum or plasma oxalate concentrations at therapeutically relevant levels or oxalate deposits.

### Treatment:

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In particular, the treatment described herein relates to blocking mRNA translation by RNAi resulting in protein expression reduction of RNAi target genes. Therefore, the surrogate parameter oxalate concentration in serum and plasma will be assessed on a weekly, monthly or quarterly basis, preferably monthly, depending on the risk profile and response in the individual patient. In the initial phase of therapy with Lumasiran/Nedosiran, the frequency of controls of oxalate concentrations might vary. The prophylactic therapy as described herein is intended to encompass prevention or reduction of oxalate related cardiovascular diseases, due to reducing oxalate concentrations by blocking oxalate relevant protein production with the compounds described herein.

### Pharmaceutical Compositions and Methods of administration

The present invention also relates to a pharmaceutical composition comprising the compounds described herein. The invention also relates to pharmaceutically acceptable salts of the compounds described herein, in addition to enantiomers and/or tautomers of the compounds described. The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" or "carrier substance" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions.

The pharmaceutical composition containing the active ingredient may be in a form suitable for oral use, for example, as tablets, chewing tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions. Tablets contain the active ingredient in admixture with non-toxic pharmaceutically acceptable excipients which are suitable for the manufacture of tablets. The tablets may be uncoated or they may be coated by known techniques to delay disintegration and absorption in the gastrointestinal tract and thereby provide a sustained action over a longer period.

The amount of active ingredient that may be combined with the carrier materials to produce a single dosage form will vary depending upon the patient treated and the particular mode of administration. For example, a formulation intended for the intravenous or subcutaneous administration of humans may vary from about 5 to about 95% of the total composition. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy. The dosage effective amount of compounds according to the invention will vary depending upon factors including the particular compound, toxicity, and inhibitory activity, the condition treated, and whether the compound is administered alone or with other therapies.

The invention relates also to a process or a method for the treatment of the mentioned pathological conditions. The compounds of the present invention can be administered prophylactically or therapeutically, preferably in an amount that is effective against the mentioned disorders, to a warm-blooded animal, for example a human, requiring such treatment, the compounds preferably being used in the form of pharmaceutical compositions.

In some embodiments, a patient may receive therapy for the treatment and/or management of the oxalate levels and/or oxalate-related cardiovascular diseases before, during or after the administration of the therapeutically effective regimen of the compound of the invention, or a pharmaceutically acceptable salt thereof. Non-limiting examples of such a therapy include dietary changes, phosphate binders, calcium or magnesium containing supplements, colestyramine, and any combination thereof. In some embodiments, the patient has not previously received a therapy for the treatment and/or management of the oxalate levels and/or oxalate-related cardiovascular diseases.

### FIGURES

The invention is demonstrated by way of the example through the figures disclosed herein. The figures provided represent particular, non-limiting embodiments and are not intended to limit the scope of the invention.

Short description of the figures:
***Figure 1******:* Oxalate concentration and its correlation with clinical parameters.**
**Figure 2****: Oxalate concentrations and the risk of all-cause mortality in 104 US patients (US cohort) with kidney failure requiring chronic dialysis.**
**Figure 3****: Oxalate concentrations and the risk of mortality/cardiovascular events in 1108 European patients (4D cohort) with kidney failure requiring chronic hemodialysis.**

Detailed description of the figures:
**Figure 1****: Oxalate concentration and its correlation with clinical parameters:** (A) In 104 kidney failure patients in the US (US cohort), plasma oxalate concentration was associated with time on dialysis (Spearman's rank correlation). (B) In 104 kidney failure patients in the US (US cohort), plasma oxalate concentration was associated with residual kidney function, defined as urine output > 300 ml/day (unpaired t-test).
**Figure 2****: Oxalate concentrations and the risk of all-cause mortality in 104 US patients (US cohort) with kidney failure requiring chronic dialysis:** (A) Kaplan-Meier curves for overall survival after a median of 2.5 years are shown in the US cohort with oxalate concentration of 30 µM as a cut-off. The vertical lines represent censored events i.e., patients who had been followed up for less than 2.5 years at the time of analysis. HR: hazard ratio; CI: confidence interval. (B) Kaplan-Meier curves for overall survival after a median of 2.5 years are shown in the US cohort with oxalate concentration of 40 µM as a cut-off. The vertical lines represent censored events i.e., patients who had been followed up for less than 2.5 years at the time of analysis. HR: hazard ratio; CI: confidence interval.
**Figure 3****: Oxalate concentrations and the risk of mortality/cardiovascular events in 1108 European patients (4D cohort) with kidney failure requiring chronic hemodialysis.** Multivariable-adjusted hazard ratios (HRs) and 95% Cis (error bars) for all-cause mortality, combined cardiovascular events, death due to heart failure and sudden cardiac death in the 4D cohort stratified by oxalate concentrations at baseline (quartiles). Models are adjusted for age, sex, time on hemodialysis, use of diuretics, C-reactive protein, body mass index, hemoglobin, albumin, previous coronary artery disease, and use of atorvastatin. CI: confidence interval.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

Elevated oxalate concentrations are a novel risk factor for all-cause mortality, death from heart failure, and sudden cardiac death in dialysis patients. These findings suggest that studies are warranted to test whether reducing dietary oxalate absorption or pharmacological measures to limit endogenous oxalate generation improve cardiovascular mortality in dialysis patients.

The examples below present oxalate concentrations as a novel risk factor for all-cause mortality, death from heart failure, and sudden cardiac death in dialysis patients. In preferred embodiments, the clinical significance of oxalate accumulation in kidney failure patients has been defined in two different cohorts, with oxalate being measured in plasma or serum. In preferred embodiments, pharmacological measures to limit endogenous oxalate generation improve cardiovascular mortality in dialysis patients.

Described in more detail below is:
- Study design and clinical characteristics of patient cohorts analyzed for all-cause death and cardiovascular events
- Significant association between oxalate concentrations above 40 µM with an increased risk of all-cause mortality
- Significant association between oxalate concentrations above 59.7 µM with an increased risk of death due to congestive heart failure and risk of sudden cardiac death.

### Example 1: Determinants of oxalate concentrations and association with mortality in the US cohort.

106 patients in the US met the inclusion criteria, of whom 104 had a plasma oxalate measurement; the baseline characteristics are summarized in **Table 1.**

**Table 1. Characteristics of 104 US patients with kidney failure requiring chronic dialysis (US study). (HD: hemodialysis; PD: peritoneal dialysis; BMI: body mass index, calculated as weight in kilograms divided by height in meters squared; AV: arteriovenous. In HD patients, Kt/V was calculated from three previous HD treatments. In PD patients, Kt/V was obtained as single measurement every three months. For analysis, just peritoneal (not urinary) Kt/V was used. Continuous variables are expressed as mean (SD) or median (IQR)* where appropriate, categorical variables as No. (%).)**

| **Characteristics** | **All patients (n=104)** | **Patients with oxalate ≥30 µM (n=22)** | **Patients with oxalate ≥40 µM (n=7)** |
|---|---|---|---|
| Oxalate [µM]* | 23.8 (10.3) range: <2-59.6 | 35.1(7.7) range: 30.1-59.6 | 45.3 (14.3) range: 40.6-59.6 |
| Age [years] | 65.9 (14.6) | 66.6 (15.5) | 66(7.9) |
| Male, No. (%) | 53 (51) | 15 (68.2) | 4 (57.1) |

| Dialysis mode, No. (%) | | | |
|---|---|---|---|
| HD | 81 (77.9) | 18 (81.8) | 6 (85.7) |
| PD | 23 (22.1) | 4 (18.2) | 1 (14.3) |
| Weight [kg] | 89.1 (25.2) | 96.8 (27.5) | 90.4 (26) |
| BMI [kg/m2] | 31.4 (8.3) | 33.8 (9.6) | 35.9 (13.4) |
| Kt/V HD patients | 1.7 (0.33) | 1.5 (0.34) | 1.5 (0.23) |
| Kt/V PD patients | 1.6 (0.53) | 2.0 (0.33) | 2.1 |
| Time on dialysis [months]* | 41.5 (52) | 51 (53) | 115 (92) |

| Urine output, No. (%) | | | |
|---|---|---|---|
| Diuresis > 300 mL/day | 28 (26.9) | 1 (4.5) | 0 |
| Diuresis < 300 mL/day | 63 (60.6) | 20 (91) | 6 (85.7) |
| Not defined | 13 (12.5) | 1 (4.5) | 1 (14.3) |

Oxalate concentrations ranged from <2 to 59.6 µM with the median being 23.8 (IQR 10.3) µM. One fifth of all patients had oxalate concentrations ≥30 µM and less than 10% had oxalate concentrations ≥40 µM. As shown in **Figure 1A****,** plasma oxalate was significantly associated with time on dialysis (r=0.30, P=0.002), but not with age, BMI or Kt/V **(Table 2).**

**Table 2: Correlation between oxalate concentration and clinical parameters in the US cohort (n=104). (Spearman's rank correlation. R: correlation coefficient. BMI: body mass index, calculated as weight in kilograms divided by height in meters squared. In HD patients, Kt/V was calculated from three previous HD treatments. In PD patients, Kt/V was obtained as single measurement every three months. For analysis, only peritoneal (not urinary) Kt/V was used.)**

| | **Oxalate concentration** | |
|---|---|---|
| **Parameter** | r | P |
| Age | 0.02 | 0.87 |
| BMI | 0.05 | 0.61 |
| Kt/V | 0.04 | 0.70 |
| Time on dialysis | 0.30 | 0.002 |

Additionally, oxalate concentration was on average almost 10 µM higher in the absence of residual kidney function. Patients with a urine output >300 mL/day demonstrated a median oxalate of 17.9 (9.5) nM; in contrast, those with a urine output <300 mL/day showed a median oxalate of 25.9 (12) µM (P<0.001, **Figure 1B****).** Adjustment for age, BMI, and Kt/V in a multivariable regression model did not change the direction, magnitude, or significance of the association between oxalate and time on dialysis as well as residual kidney function **(Table 3).**

**Table 3: Multivariable regression of oxalate concentration, age, BMI, Kt/V, time on dialysis, and residual kidney function in the US cohort (n=104). (Dependent variable: oxalate concentration; covariates: age, BMI, Kt/V, time on dialysis, and residual kidney function; time on dialysis was transformed to its natural logarithm to obtain normally distributed values; categorical variable: residual kidney function, defined as urine output >300 mL/day. BMI: body mass index, calculated as weight in kilograms divided by height in meters squared. In HD patients, Kt/V was calculated from three previous HD treatments. In PD patients, Kt/V was obtained as single measurement every three months. For analysis, only peritoneal (not urinary) Kt/V was used.)**

| | **Oxalate concentration** | |
|---|---|---|
| **Covariates** | r | P |
| Age | 0.06 | 0.63 |
| BMI | 0.12 | 0.44 |
| Kt/V | -0.20 | 0.15 |
| Ln(time on dialysis) | 0.29 | 0.01 |
| Residual kidney function | -0.36 | 0.002 |

After a follow-up of 2.5 years, 15 patients had deceased. By regression analyses, patients with oxalate concentrations ≥30 µM had a higher mortality than patients below this cut-off (HR 2.9, 95% Cl 1.02-8.04, P=0.04, **Figure 2A****).** Patients with oxalate concentrations ≥40 µM had a more than 4-fold increased mortality compared to patients with oxalate concentrations <40 µM (HR 4.7, 95% Cl 1.32-16.70, P=0.008, **Figure 2B****).**

### Example 2: Oxalate concentrations and clinical outcomes in the 4D Study

A total of 1108 patients had baseline oxalate measurements. The median follow-up was 4.0 years in the atorvastatin arm and 4.1 years in the placebo arm. During follow-up, 548 patients died, including 139 (25.4%) patients who died from sudden cardiac death and 38 (6.9%) who died due to congestive heart failure. A 1 total of 413 patients reached the primary composite cardiovascular endpoint with myocardial infarction (fatal or non-fatal) and stroke (fatal or non-fatal) occurring in 177 and 90 patients, respectively. The baseline patient characteristics are shown in **Table 4.** The median (IQR) oxalate concentration at baseline was 42.4 (30) µM with no significant difference between the atorvastatin and placebo group. Patients with oxalate concentrations in the highest quartile were younger, more likely to be female, had higher serum albumin, higher high-density lipoprotein cholesterol, higher serum phosphate, higher leukocyte counts, and higher serum creatinine **(Table 4).** In addition, as observed in the US cohort, higher oxalate concentrations were positively associated with longer time on HD (P<0.001). Furthermore, there was a strong correlation between serum oxalate concentration and NT-proBNP (P=0.001) concentrations.

**Table 4. Characteristics of 1108 European patients requiring chronic hemodialysis stratified by quartiles of oxalate concentration at baseline (4D Study). (Continuous variables are expressed as mean (SD) or median (IQR)* where appropriate, categorical variables as No. (%). † P-value of ANOVA F statistic (for continuous outcomes) or Pearson chi square statistic (for categorical outcomes).**

| **Characteristics** | **Quartile 1 ≤29.6 µM n=274** | **Quartile 2 29.7-42.3 µM n=279** | **Quartile 3 42.4-59.6 µM n=274** | **Quartile 4 ≥59.7 µM n=281** | **P-value†** |
|---|---|---|---|---|---|
| Age [years] | 67.6 (8.1) | 66.1 (8.3) | 66.1 (8.1) | 65.6 (8.5) | 0.03 |
| Male, No. (%) | 159 (58) | 153 (55) | 148 (54) | 143 (51) | 0.41 |
| Atorvastatin treatment, No. (%) | 132 (48) | 140 (50) | 137 (50) | 135 (48) | 0.95 |
| Cholesterol total [mg/dL] | 215 (41) | 217 (44) | 217 (40) | 217 (36) | 0.91 |
| Smoking, No. (%) | 30 (11) | 22 (8) | 22 (8) | 25 (9) | 0.43 |
| Systolic blood pressure [mmHg] | 143 (23) | 146 (21) | 145 (21) | 147 (23) | 0.08 |
| Diastolic blood pressure [mmHg] | 76 (11) | 75 (10) | 77 (11) | 76 (12) | 0.54 |
| Body mass index [kg/m²] | 27.6 (4.4) | 27.8 (5.2) | 27.0 (4.3) | 27.2 (5.0) | 0.20 |
| CRP* [µg/mL] | 5.2 (8.5) | 6.1 (8.8) | 6.5 (8.3) | 7.6 (8.6) | 0.47 |
| Duration of diabetes [years] | 18.3 (9.1) | 17.9 (8.3) | 17.8 (8.5) | 18.4 (8.4) | 0.81 |
| Time on hemodialysis [months] | 6.4 (6.0) | 7.4 (6.2) | 9.1 (7.4) | 9.8 (7.2) | <0.001 |
| NT-proBNP [pg/mL] | 5771.6 | 7760.5 | 9218.4 | 17745.0 | 0.001 |
| Coronary artery disease, No. (%) | 85 (31) | 75 (27) | 85 (31) | 79 (28) | 0.64 |
| Use of diuretics, No. (%) | 227 (83) | 223 (80) | 206 (75) | 211 (75) | 0.60 |

### Example 3: Oxalate and mortality risk

The pattern we observed was similar as in the US cohort with a tendency towards an increased risk of all-cause death in patients with increased oxalate concentrations. As shown in **Table 5,** patients in the highest oxalate quartile had a 23% increased risk of death, however, the lower bound of the 95% CI did cross below 1 (HR 1.23, 95% CI 0.97-1.56, P=0.08).

**Table 5. Risk of all-cause mortality, combined cardiovascular events, death due to heart failure, sudden cardiac death, myocardial infarction, and stroke, stratified by quartiles of oxalate concentration at baseline (4D Study, n=1108). HR: hazard ratio; 95% CI: 95% confidence interval. (* Adjusted HR: adjustments were made for age, sex, time on hemodialysis, use of diuretics, and use of atorvastatin. † Adjusted HR: adjustments were made for age, sex, time on hemodialysis, use of diuretics, C-reactive protein, body mass index, hemoglobin, albumin, previous coronary artery disease, and use of atorvastatin. ‡ Combined cardiovascular events were defined as a composite of death from cardiac causes, fatal or nonfatal stroke, and nonfatal myocardial infarction, whichever occurred first. Death from cardiac causes comprised death due to congestive heart failure, sudden cardiac death, fatal myocardial infarction, death due to coronary artery disease during or within 28 days after an intervention, and all other deaths attributable to coronary artery disease.)**

| | **HRs stratified by oxalate quartiles at baseline** | | | | |
|---|---|---|---|---|---|
| **Outcome** | **Quartile 1 ≤29.6 µM n=274** | **Quartile 2 29.7-42.3 µM n=279** | **Quartile 3 42.4-59.6 µM n=274** | **Quartile 4 ≥59.7 µM n=281** | **Global P-value** |
| **All-cause** mortality | | | | | |
| Crude HR (95% CI) | 1 | 1.01 (0.78-1.30) | 1.02 (0.81-1.29) | 1.06 (0.83-1.35) | 0.63 |
| Adjusted* HR (95% CI) | 1 | 1.06 (0.81-1.39) | 1.11 (0.88-1.42) | 1.17 (0.92-1.50) | 0.19 |
| Adjusted^{†} HR (95% CI) | 1 | 1.05 (0.80-1.38) | 1.16 (0.91-1.47) | 1.23 (0.97-1.56) | 0.08 |

| **Cardiovascular events‡** | | | | | |
|---|---|---|---|---|---|
| Crude HR (95% CI) | 1 | 0.90 (0.67-1.20) | 0.92 (0.70-1.20) | 1.25 (0.98-1.59) | 0.07 |
| Adjusted* HR (95% CI) | 1 | 0.92 (0.69-1.24) | 0.97 (0.74-1.28) | 1.33 (1.04-1.72) | 0.03 |
| Adjusted^{†} HR (95% CI) | 1 | 0.94 (0.70-1.27) | 1.01 (0.76-1.33) | 1.40 (1.08-1.81) | 0.01 |

| **Death due to heart failure** | | | | | |
|---|---|---|---|---|---|
| Crude HR (95% CI) | 1 | 1.01 (0.37-2.78) | 1.21 (0.46-3.23) | 1.77 (0.69-4.55) | 0.24 |
| Adjusted* HR (95% CI) | 1 | 1.12 (0.40-3.12) | 1.48 (0.57-3.87) | 2.31 (0.91-5.83) | 0.08 |
| Adjusted^{†} HR (95% CI) | 1 | 1.07 (0.37-3.07) | 1.47 (0.57-3.83) | 2.40 (0.99-5.80) | 0.05 |

| **Sudden cardiac death** | | | | | |
|---|---|---|---|---|---|
| Crude HR (95% CI) | 1 | 1.18 (0.74-1.88) | 1.34 (0.83-2.18) | 1.39 (0.91-2.12) | 0.13 |
| Adjusted* HR (95% CI) | 1 | 1.23 (0.77-1.97) | 1.45 (0.88-2.39) | 1.50 (0.96-2.34) | 0.07 |
| Adjusted^{†} HR (95% CI) | 1 | 1.26 (0.76-2.07) | 1.50 (0.89-2.55) | 1.62 (1.03-2.56) | 0.04 |

| **Myocardial infarction** | | | | | |
|---|---|---|---|---|---|
| Crude HR (95% CI) | 1 | 0.98 (0.641.51) | 0.88 (0.58-1.38) | 1.28 (0.88-1.86) | 0.20 |
| Adjusted* HR (95% CI) | 1 | 1.00 (0.65-1.54) | 0.91 (0.60-1.38) | 1.29 (0.88-1.89) | 0.19 |
| Adjusted^{†} HR (95% CI) | 1 | 1.03 (0.67-1.57) | 0.94 (0.62-1.42) | 1.34 (0.91-1.98) | 0.14 |

| **Stroke** | | | | | |
|---|---|---|---|---|---|
| Crude HR (95% CI) | 1 | 0.55 (0.30-1.01) | 0.63 (0.36-1.10) | 1.08 (0.67-1.73) | 0.75 |
| Adjusted* HR (95% CI) | 1 | 0.55 (0.30-1.00) | 0.64 (0.35-1.15) | 1.12 (0.69-1.82) | 0.66 |
| Adjusted^{†} HR (95% CI) | 1 | 0.54 (0.29-0.99) | 0.68 (0.38-1.22) | 1.19 (0.71-2.00) | 0.50 |

### Example 4: Combined cardiovascular events, death due to heart failure and sudden cardiac death

The incidence of combined cardiovascular events, the primary composite outcome of the 4D Study, was markedly higher at higher oxalate concentrations **(****Figure 3****).** Patients in the highest oxalate quartile had a 40% increased risk to reach the combined endpoint after adjustment for confounders including dialysis vintage and residual renal function identified in the US cohort (adjusted HR 1.40, 95% CI 1.08-1.81, P=0.01; **Table 5).**

Higher baseline oxalate concentrations were also associated with incrementally higher risk of death due to congestive heart failure. The patients in the highest quartile were more than 2-fold more likely to die of heart failure than patients in the lowest quartile (adjusted HR 2.40, 95% CI 0.99-5.80), as shown in **Table 5** and **Figure 3****.** The risk of sudden cardiac death as a separate outcome measure was found to increase incrementally and significantly with higher oxalate quartiles. Patients in the highest quartile exhibited a 62% higher sudden death risk (adjusted HR 1.62, 95% CI 1.03-2.56, P=0.04) compared to those in the lowest quartile **(****Figure 3****).** The observed increase of NT-proBNP levels over oxalate quartiles suggested a potential intermediate pathway. When we additionally adjusted our analyses for NT-proBNP, there was a mild attenuation of the association (adjusted HR 1.51, 95% CI 0.94-2.43) for the highest compared to the lowest quartile. Associations between serum oxalate and myocardial infarction and stroke individually were consistent with the overall findings for the composite events, heart failure and sudden cardiac death, but the associations were weaker and were not statistically significant alone.

### Material and Methods used for Examples 1-4

### Study Design and Participants

The present study comprises two patient cohorts: an observational dialysis cohort in the US (hemodialysis, HD and peritoneal dialysis, PD) and the German Diabetes and Dialysis Study (4D), a prospective trial cohort (only HD). In the US, 106 patients, aged ≥18 years, were recruited from four outpatient dialysis centers in Connecticut for an observational study with mortality as primary endpoint. Participants received either thrice weekly HD for 3-5 hours per treatment session or daily home PD. Patients eligible for this study had to be (1) on HD or PD treatment and (2) medically stable with no infections or hospitalizations for a minimum of 3 months. HD patients had to have (3) an access blood flow of ≥250 mL/min and (4) a dialysate flow of ≥500 mL/min. Between April and September 2016, one-time blood samples were collected from all patients to measure plasma oxalate concentrations. Patients were followed until the date of death, transplantation, or end of the study in November 2018. Local authorities (Western Institutional Review Board Study No. 1162867) approved the study and all patients gave their written informed consent before inclusion. Design and methods of the 4D Study have been previously reported.⁷ In brief, the 4D Study was a prospective randomized controlled trial including 1255 patients with type 2 diabetes mellitus, aged 18-80 years, who had begun HD within the previous 2 years. Between March 1998 and October 2002, patients were recruited in 178 dialysis centers in Europe and randomly assigned to double-blind treatment with 20 mg atorvastatin (n=619) or placebo (n=636) once daily. At each follow-up every 6 months until March 2004, blood samples were taken and clinical information was recorded, including any adverse events and an electrocardiogram. The study conforms with the principles outlined in the Declaration of Helsinki. It was approved by the medical ethics committee of the University of Würzburg, Germany, and all patients gave their written informed consent before inclusion.

### Data collection

In the US cohort, clinical, supplementary laboratory, and mortality data were collected from electronic health records and complemented by reports from the treating physician assistants. Follow-up was thrice weekly for HD and once a month for PD patients, according to standard clinical routines. Single pool Kt/V (spKt/V) was calculated using the second generation Daugirdas formula.⁸ Mean spKt/V was calculated from three previous HD treatments. In PD patients, Kt/V (peritoneal and urinary) were obtained as single measurements every three months. In the 4D cohort, demographic and clinical information was obtained through patient interviews and reports from the treating nephrologists. Coronary artery disease was defined by a history of myocardial infarction, coronary artery bypass grafting surgery, percutaneous coronary intervention, or the presence of typical vascular findings by coronary angiography.

### Outcome assessment

In the US cohort, all-cause mortality was the primary endpoint. In the 4D Study, the primary endpoint was a composite of cardiac death, nonfatal myocardial infarction, and fatal or nonfatal stroke, whichever occurred first (composite cardiovascular endpoint). Death from cardiac causes comprised: death due to congestive heart failure, sudden cardiac death, fatal myocardial infarction, death due to coronary disease during or within 28 days after an intervention, and all other deaths attributable to coronary artery disease. Sudden cardiac death was defined as: death verified by terminal rhythm disorders in an electrocardiogram, death observed by witnesses within one hour after the onset of cardiac symptoms, sudden cardiac death confirmed by autopsy, or unexpected death presumably or possibly of cardiac origin and in absence of a potassium level ≥7.5 mmol/L before the start of the three most recent HD sessions. Myocardial infarction was diagnosed when two of the following three criteria were met: typical symptoms, elevated levels of cardiac enzymes (i.e., a level of creatine kinase MB >5% of the total level of creatine kinase, a level of lactic dehydrogenase 1.5 times the upper limit of normal, or a level of troponin T >2 ng/mL), or diagnostic changes on the electrocardiogram. Stroke was defined as a neurologic deficit lasting >24 hours. Computed tomographic or magnetic resonance imaging was available in all but 16 cases. The 4D Study endpoints were centrally adjudicated and categorized by three members of the endpoint adjudication committee blinded to study treatment and according to pre-defined criteria.⁷ For the present analysis, all-cause mortality, composite cardiovascular endpoint, death due to congestive heart failure, sudden cardiac death, myocardial infarction (fatal and nonfatal), and stroke (fatal and nonfatal), were chosen as separate outcome measures, and thus include the core cardiovascular outcome measures as suggested by the Standardised Outcomes in Nephrology-Hemodialysis initiative.⁹

### Sample handling and oxalate measurement

In the US cohort, oxalate in HD and PD patients was measured in EDTA plasma samples obtained prior to dialysis therapy at the first appointment after a long interval and at the monthly clinic appointment, respectively. As previously described, samples were immediately put on ice, centrifuged, and filtered, and the filtrate was acidified with 20 µL 1N hydrochloric acid per 500 µL of plasma, aliquoted, and stored at -80°C within 2 hours.24 The frozen and acidified filtrate was thawed, and oxalate was measured enzymatically using oxalate oxidase (Trinity Biotech; Bray, Co. Wicklow, Ireland).^{10,11} The lower limit of detection of our assay was 2 µM. To evaluate the validity of one-time sampling, we collected repeat blood samples from 11 HD patients (weekly over 2-4 consecutive weeks) and 13 PD patients (monthly over 2-4 consecutive months). The intraindividual variability (median CV) was 9.4% in the HD and 16.7% in the PD group. In the 4D cohort, oxalate concentrations were measured in baseline serum samples taken 1 week prior to randomization before the start of dialysis sessions and administration of drugs, and stored at - 80°C. Frozen serum samples were slowly thawed, vigorously vortexed, deproteinized, acidified, and measured as previously reported.¹² The median CV for repeated measurements in the stored eluate of 36 randomly selected samples was 4.1%. Sodium oxalate solutions (10 µM and 50 µM) and pooled patient samples at 15 and 30 µM oxalate concentration were used as quality controls.

### Statistical Analyses

We calculated means (SDs) or medians (interquartile ranges [IQRs]) for continuous and frequency tables for categorical variables. We compared characteristics between groups by Student's t-tests, analyses of variation (ANOVA), Mann-Whitney U tests, or chi-square tests where appropriate. In the US cohort, we investigated clinical determinants of oxalate by Spearman correlation and regression modeling. After a follow-up of 2.5 years, we analyzed survival stratified by oxalate concentration below or above 30 or 40 µM at baseline, using the Kaplan-Meier method and log-rank test. In the 4D cohort, patient characteristics are presented in subgroups defined by quartiles of oxalate concentrations at baseline, with the following cut-points: ≤29.6 µM, >29.6 to ≤42.3 µM, >42.3 to ≤59.6 µM, and >59.6 µM. The risk of all-cause mortality, reaching the composite cardiovascular endpoint, death due to congestive heart failure, sudden cardiac death, myocardial infarction, and stroke according to oxalate quartiles (with quartile 1 being the reference group) were assessed by Cox regression models. First, we fitted a model including the main confounding variables i.e., age, sex, time on HD, and use of diuretics (model 1). Second, we fitted a model additionally adjusting for other risk factors of mortality in kidney failure as recently identified by a meta- analysis,¹³ including C-reactive protein (CRP), BMI, hemoglobin, albumin, and previous coronary artery disease (model 2 - core model). To explore potential mediating pathways, we calculated another model including NT-proBNP (model 3 - mediating model). All models were adjusted for treatment group (atorvastatin vs. placebo) and fit as complete case analyses (no missing value imputation). P-values are two-sided. Statistical analyses were conducted using IBM SPSS Statistics, Version 25 (IBM Corp; Armonk, NY, USA) for the US study, and STATA (StataCorp. 2017. Stata Statistical Software: Release 15. College Station, TX: StataCorp LLC) for the 4D Study.

### References

1 Ermer, T., Eckardt, K. U., Aronson, P. S. & Knauf, F. Oxalate, inflammasome, and progression of kidney disease. Curr Opin Nephrol Hypertens 25, 363-371, (2016).
2 Hoppe, B. An update on primary hyperoxaluria. Nature reviews. Nephrology 8, 467-475, doi:10.1038/nrneph.2012.113 (2012).
3 Weigert, A., Martin-Higueras, C. & Hoppe, B. Novel therapeutic approaches in primary hyperoxaluria. Expert opinion on emerging drugs, 1-9, (2018).
4 https://www.businesswire.com/news/home/20200331005129/en/Dicerna-Initial-Observations-PHYOXTM3-Trial-Nedosiran-Treatment, March 2020).
5 Knauf, F. et al. NALP3-mediated inflammation is a principal cause of progressive renal failure in oxalate nephropathy. Kidney Int 84, 895-901, doi:10.1038/ki.2013.207 (2013).
6 Waikar, S. S. et al. Association of Urinary Oxalate Excretion With the Risk of Chronic Kidney Disease Progression. JAMA Intern Med, doi:10.1001/jamainternmed.2018.7980 (2019).
7 Wanner C, Krane V, Marz W, Olschewski M, Mann JF, Ruf G, Ritz E, German D and Dialysis Study I. Atorvastatin in patients with type 2 diabetes mellitus undergoing hemodialysis. The New England 19 journal of medicine. 2005;353:238-48.
8 Daugirdas JT. Second generation logarithmic estimates of single-pool variable volume Kt/V: an analysis of error. Journal of the American Society of Nephrology : JASN. 1993;4:1205-13.
9 O'Lone E, Viecelli AK, Craig JC, Tong A, Sautenet B, Herrington WG, Herzog CA, Jafar TH, Jardine M, Krane V, Levin A, Malyszko J, Rocco MV, Strippoli G, Tonelli M, Wang AYM, Wanner C, Zannad F, Winkelmayer WC, Wheeler DC and Investigators S-HCCW. Establishing Core Cardiovascular Outcome Measures for Trials in Hemodialysis: Report of an International Consensus Workshop. American journal of kidney diseases : the official journal of the National Kidney Foundation. 2020;76:109-120.
10 Ermer T, Kopp C, Asplin JR, Granja I, Perazella MA, Reichel M, Nolin TD, Eckardt K-U, Aronson PS, Finkelstein FO and Knauf F. Impact of Regular or Extended Hemodialysis and Hemodialfiltration on Plasma Oxalate Concentrations in Patients With End-Stage Renal Disease. Kidney International Reports. 2017;2:1050-1058.
11 Ladwig PM, Liedtke RR, Larson TS and Lieske JC. Sensitive spectrophotometric assay for plasma oxalate. Clinical chemistry. 2005;51:2377-80.
12 Pfau A, Wytopil M, Chauhan K, Reichel M, Coca S, Aronson P, Eckardt KU, Knauf F. Assessment of Plasma Oxalate Concentration in Patients With CKD. Kidney international reports, 5: 2013-2020, 2020 10.1016/j.ekir.2020.08.029.
13 Ma L and Zhao S. Risk factors for mortality in patients undergoing hemodialysis: A systematic review and meta-analysis. International journal of cardiology. 2017;238:151-158.

## Claims

1. A composition comprising Lumasiran and/or Nedosiran for use in the treatment or prevention of an oxalate-related disorder in a patient receiving dialysis treatment who does not suffer from Primary Hyperoxaluria 1-3 (PH).

2. The composition comprising Lumasiran and/or Nedosiran for use according to claim 1, wherein said oxalate-related disorders comprise cardiovascular disease in a non-PH patient receiving dialysis treatment.

3. The composition comprising Lumasiran and/or Nedosiran for use according to any of the preceding claims, wherein said oxalate-related cardiovascular disease is associated with calcium oxalate deposition in organs in a non-PH patient receiving dialysis treatment.

4. The composition comprising Lumasiran and/or Nedosiran for use according to claim 1 to 3, wherein said oxalate-related cardiovascular disease is associated with plasma or serum oxalate concentrations >20µM, preferably >30µM, in a non-PH patient receiving dialysis treatment.

5. The composition comprising Lumasiran and/or Nedosiran for use according to claim 4, wherein said oxalate concentrations are associated with cardiovascular events, preferably sudden cardiac death and congestive heart failure in a non-PH patient receiving dialysis treatment.

6. The composition comprising Lumasiran and/or Nedosiran for use according to any of the preceding claims, wherein said composition reduces hepatic oxalate production and thus lowers plasma or serum oxalate concentrations in a non-PH patient receiving dialysis treatment.

7. The composition comprising Lumasiran and/or Nedosiran for use according to any of the preceding claims, wherein said composition is administered subcutaneously, intravenously or orally at therapeutically relevant dosage at the time of dialysis treatment.

8. The composition comprising Lumasiran and/or Nedosiran for use according to claim 7, wherein said administered dosage and frequency is suitable to achieve a plasma or serum oxalate concentration <30µM and/or lower as before treatment initiation, preferably a plasma or serum oxalate concentration at least 20% lower as before treatment initiation.

9. The composition comprising Lumasiran and/or Nedosiran for use according to claim 2 to 8, wherein administration of said composition prevents or reduces vascular calcification at therapeutically relevant levels caused by calcium oxalate.

10. A composition comprising the nucleic acid, dsNA, or hybridization complex of Lumasiran and/or Nedosiran or its salts thereof, wherein said nucleic acid, dsNA, or hybridization complex is present in an amount effective to reduce cytosolic mRNA levels of its target genes when introduced into a cell of a non-PH patient receiving dialysis treatment.

11. The composition according to claim 10, wherein said nucleic acid, dsNA, or hybridization complex is preferably coated to a pharmaceutically acceptable carrier and formulated in a pharmaceutically buffered solution.

12. A method for preparing the composition comprising Lumasiran and/or Nedosiran according to claim 10 and 11.

13. A kit comprising Lumasiran and/or Nedosiran according to any of the preceding claims, and instructions for its use.
